# DEMANDE DE BREVET EUROPEEN

(11) **EP 1 230 913 A2**
(43) Date de publication de la demande: **14.08.2002**
(21) Numéro de dépôt: 02290091.4
(22) Date de dépôt: 15.01.2002
(51) Int. Cl.: A61K 7/48

(54) **Utilisation cosmétique d'un complexe de photoprotecteurs cellulaires comme agent anti-pollution**

(30) Priorité: 07.02.2001 FR 0101643
(71) Demandeur: L'OREAL, 75008 Paris (FR)
(72) Inventeur: Pelletier, Pacale, 92160 Antony (FR); Catroux, Philippe, 94130 Nogent sur Marne (FR)
(74) Mandataire: Renard, Emmanuelle

(57) **Abrégé**

La présente demande se rapporte à l'utilisation en application topique, d'un complexe de photoprotecteurs cellulaires comme agent anti-pollution, notamment comme agent cosmétique anti-pollution.

La demande se rapporte aussi à un procédé de traitement cosmétique en vue de protéger l'organisme contre les effets de la pollution, consistant à appliquer sur la matière kératinique, une composition contenant dans un milieu physiologiquement acceptable, une quantité efficace d'un complexe de photoprotecteurs cellulaires.

## Description

La présente demande se rapporte à l'utilisation en application topique, d'un complexe de photoprotecteurs cellulaires comme agent anti-pollution, et à un procédé de traitement cosmétique en vue de protéger l'organisme contre les effets de la pollution, consistant à appliquer sur la matière kératinique, une composition contenant dans un milieu physiologiquement acceptable, une quantité efficace d'un complexe de photoprotecteurs cellulaires.

Les milieux urbains sont régulièrement soumis à des pics de pollution. L'individu dans son environnement quotidien et particulièrement en zone urbaine, peut être soumis à de multiples agressions au niveau des matières kératiniques, et notamment de la peau, du cuir chevelu et des cheveux, par différents polluants aériens. Les polluants atmosphériques qui sont représentés largement par les produits primaires et secondaires de la combustion représentent une source importante du stress oxydatif environnemental. Différents types de produits chimiques, xénobiotiques et particules, composent la pollution urbaine. Trois grandes catégories de polluants peuvent exercer des effets délétères sur la peau et le cheveu : les gaz, les métaux lourds et les particules qui sont des résidus de combustion sur lesquelles sont absorbés de très nombreux composés organiques.

Ce sont les tissus les plus externes qui sont initialement et directement exposés aux toxiques de l'environnement. La peau est directement et fréquemment exposée à l'environnement pro-oxydant. Elle est particulièrement sensible à l'action du stress oxydatif, et sa couche la plus externe sert de barrière vis-à-vis des dommages oxydatifs. Dans la plupart des circonstances, l'oxydant a des chances d'être neutralisé après réaction avec les matières kératiniques, mais les produits de réaction formés peuvent être responsables d'atteintes cellulaires et tissulaires. Le stratum corneum, la barrière de la peau, est le site de contact entre air et tissu cutané. La structure biphasique lipides/protéines est un déterminant crucial de cette fonction de barrière de la peau. Ces éléments peuvent réagir avec les oxydants et être altérés ce qui favorisera les phénomènes de desquamation.
Parmi les gaz polluants aériens urbains, on trouve l'ozone troposphérique dont la formation résulte de l'interaction entre les hydrocarbures polycycliques aromatiques, les oxydes d'azote, l'air et le rayonnement UV (Free Radical Biology Medecine, 1990, 9, 245). Ce polluant photo oxydant est connu pour réagir avec différentes cibles biologiques (lipides, protéines) localisées à la surface cutanée (sébum, stratum corneum).
La peroxydation lipidique induite par l'ozone peut altérer la peau de deux façons :
1/ l'oxydation et la dégradation des lipides du stratum corneum peut altérer la fonction barrière du stratum corneum. La perturbation des lipides externes et de l'architecture des protéines semblent être des facteurs déclenchants dans de nombreuses dermatoses (psoriasis, dermatite atopique, dermatite irritante).
2/ la formation accrue de produits d'oxydation des lipides dans les couches de peau supérieures peut déclencher des atteintes dans les couches cutanées adjacentes. La réaction de l'ozone (O₃) avec les lipides insaturés implique des réactions d'addition sur les doubles liaisons. Ce processus conduit dans un deuxième temps à la cassure des chaînes lipidiques et à la formation d'hydroperoxydes d'aldéhydes et de peroxyde d'hydrogène. Il s'agit d'un mécanisme spécifique différent du mécanisme de lipoperoxydation classiquement décrit, lequel est médié par un radical. Les produits d'oxydation lipidique secondaires ou tertiaires induits par l'ozone, qui ont une réactivité inférieure à l'ozone mais une durée de vie supérieure, peuvent propager l'effet de l'ozone. Du fait de leur stabilité relative, les produits d'oxydation des lipides et de peroxydation, c'est-à-dire les aldéhydes et les oxydes de cholestérol, ont le potentiel d'altérer les cellules à des sites distants non directement exposés à l'ozone. Une atteinte oxydative significative au niveau des couches superficielles du stratum peut initier des processus inflammatoires localisés sous-jacents, conduisant au recrutement de phagocytes qui en générant des oxydants vont amplifier les processus oxydatifs initiaux.

Dans la pollution urbaine, l'exposition concomitante à l'ozone et aux UV peut causer un stress oxydatif synergique. De même, on peut penser qu'il existe une synergie d'action entre l'ozone et les composés organiques issus de la combustion.

Parmi les polluants pouvant exercer des effets délétères sur les matières kératiniques, les gaz toxiques tels que l'ozone, le monoxyde de carbone, les oxydes d'azote ou les oxydes de soufre, sont des constituants majeurs des polluants. Il a été constaté que ces gaz toxiques favorisent la desquamation des matières kératiniques, "fatiguent" lesdites matières kératiniques, c'est-à-dire les rendent sales et ternes. De même, il a été constaté une asphyxie cellulaire des dites matières kératiniques.

Ainsi, les effets nocifs de la pollution sur les matières kératiniques affectent la respiration cellulaire de ces matières kératiniques et se traduisent par un vieillissement accéléré de la peau, avec un teint terne et la formation précoce de rides ou ridules, et aussi par une diminution de la vigueur des cheveux qui prennent aussi un aspect terne. En outre, du fait de la pollution, peau et cheveux se salissent plus rapidement. De plus, la pollution peut provoquer des irritations et des phénomènes d'allergie et d'inflammation sur la peau.

Pour lutter contre ces effets des polluants, divers agents anti-pollution ont été décrits. Ainsi le document EP-A-577 718 décrit l'utilisation des sphingolipides pour protéger la peau et les cheveux de la pollution atmosphérique.

Avec l'augmentation de la pollution, il subsiste le besoin de trouver d'autres agents permettant de lutter efficacement contre l'effet néfaste des polluants sur les matières kératiniques et empêcher l'adhérence de ces polluants sur les matières kératiniques, notamment pour éviter la dégradation de la respiration cellulaire, la desquamation et le vieillissement accéléré des matières kératiniques et notamment de la peau, ainsi que lutter contre le teint terne et la formation précoce de rides ou ridules sur la peau, pour éviter que les cheveux aient un aspect terne et se salissent, et pour éviter l'irritation de la peau ainsi que les phénomènes d'allergie cutanée et d'inflammation de la peau.

La demanderesse a maintenant trouvé, de façon tout à fait surprenante, que l'utilisation d'un complexe de photoprotecteurs cellulaires, permettait de protéger les matières kératiniques des effets des polluants.

Certes, il est connu d'utiliser les complexes de photoprotecteurs cellulaires dans des compositions à application topique et notamment cosmétiques destinées à être appliquées sur la peau, par exemple dans le but de protéger les cellules constitutives, fonctionnelles de la peau (en particulier des cellules de Langerhans) contre les rayonnements UV (voir par exemple FR-2 634 374 et Cosmetics & Toiletries, 1996, 111, 47). Toutefois, aucun document ne décrit que ces composés puissent avoir des propriétés de protection des matières kératiniques contre la pollution.

Ainsi, l'invention a pour objet l'utilisation cosmétique d'un complexe de photoprotecteurs cellulaires constitué d'un mélange comprenant au moins un acide aminé et au moins un nucléoside et/ou un nucléotide, comme agent cosmétique anti-pollution, dans une composition pour application topique sur les matières kératiniques.

La présente invention concerne également l'utilisation d'un complexe de photoprotecteurs cellulaires constitué d'un mélange comprenant au moins un acide aminé et au moins un nucléoside et/ou un nucléotide, pour la préparation d'une composition à application topique destinée à protéger les matières kératiniques contre les effets nocifs de la pollution.

Par complexe de photoprotecteurs cellulaires on entend un mélange comprenant au moins un acide aminé et au moins un nucléoside et/ou un nucléotide, le complexe pouvant être d'origine naturelle, synthétique et/ou biotechnologique en fonction de la nature de ses constituants.

Dans un aspect avantageux de l'invention, le complexe de photoprotecteurs cellulaires utilisé sera d'origine naturelle.

Un complexe de photoprotecteurs cellulaires convenant particulièrement bien à la mise en oeuvre de la présente invention est le produit commercialisé par les Laboratoires Sérobiologiques sous le nom de Photonyl. Ce produit est comprend notamment un mélange d'arginine, de mannitol, de chlorhydrate de pyridoxine, de chlorhydrate d'histidine, d'ARN hydrolysé, de triphosphate d'adénosine et de sodium, de phénylalanine, et de tyrosine.

On entend par « agent anti-pollution » un agent qui protège la peau et les matières kératiniques de façon à prévenir, atténuer et/ou supprimer les effets délétères des gaz toxiques tels que l'ozone.

Dans le cadre de la présente invention, on entend par « matière kératinique » la peau, le cuir chevelu, les cheveux, les cils, les sourcils, les ongles et les muqueuses.

On entend ici par « application topique » une application externe sur les matières kératiniques, que sont notamment la peau, le cuir chevelu, les cils, les sourcils, les ongles et les muqueuses.

La composition utilisée selon l'invention est destinée à une application topique et contient donc un milieu physiologiquement acceptable, c'est-à-dire compatible avec les tissus cutanés tels que la peau, le cuir chevelu, les cils, les sourcils, les ongles et les muqueuses. Ainsi, la composition peut être appliquée sur tout le corps humain.

Le complexe de photoprotecteurs cellulaires naturels, utilisé comme agent anti-pollution selon l'invention est avantageusement en une quantité suffisante. On entend ici par « quantité suffisante » (ou quantité efficace), une quantité telle que la protection contre les polluants soit assurée. Cette quantité peut aller par exemple de 0,01 à 10 % en poids et de préférence de 0,05 à 2 % en poids de matière active de composé anti-pollution par rapport au poids total de la composition.

Les compositions à application topique, et notamment cosmétiques, utilisées selon l'invention contiennent un milieu physiologiquement acceptable, c'est-à-dire un milieu compatible avec la peau, les lèvres, le cuir chevelu, les cils, les yeux, les ongles et/ou les cheveux. Ce milieu physiologiquement acceptable peut être plus particulièrement constitué d'eau et éventuellement d'un solvant organique physiologiquement acceptable choisi par exemple parmi les alcools inférieurs comportant de 1 à 8 atomes de carbone et en particulier 1 à 6 atomes de carbone, comme l'éthanol, l'isopropanol, le propanol, le butanol ; les polyéthylène glycols ayant de 6 à 80 oxydes d'éthylène ; les polyols comme le propylène glycol, l'isoprène glycol, le butylène glycol, la glycérine, le sorbitol. Ce peut être aussi un milieu anhydre, notamment un milieu huileux contenant des huiles et/ou matières grasses autres que les huiles.

Quand le milieu physiologiquement acceptable est un milieu aqueux, il a un pH compatible avec la peau, allant de préférence de 3 à 8 et mieux de 4 à 7.

Quand la composition comporte un milieu aqueux ou hydroalcoolique, il est possible d'ajouter une phase grasse (ou huileuse) dans ce milieu, afin que les compositions de l'invention soient plus douces et plus nourrissantes.

Ainsi, les compositions selon l'invention contenant les agents anti-pollution tels que définis ci-dessus peuvent se présenter sous toutes les formes galéniques classiquement utilisées pour une application topique et notamment sous forme de solutions aqueuses, hydroalcooliques ou huileuses, d'émulsions huile-dans-eau (H/E) ou eau-dans-huile (E/H) ou multiple (triple : E/H/E ou H/E/H), de gels aqueux ou huileux, de produits anhydres liquides, pâteux ou solides, ou de dispersions d'une phase grasse dans une phase aqueuse à l'aide de sphérules, ces sphérules pouvant être des nanoparticules polymériques telles que les nanosphères et les nanocapsules, ou des vésicules lipidiques de type ionique et/ou non ionique. Ces compositions sont préparées selon les méthodes usuelles.

En outre, les compositions utilisées selon l'invention peuvent être plus ou moins fluides et avoir l'aspect d'une crème blanche ou colorée, d'une pommade, d'un lait, d'une lotion, d'un sérum, d'une pâte, d'une mousse. Elles peuvent être éventuellement appliquées sur la peau sous forme d'aérosol. Elles peuvent aussi se présenter sous forme solide, et par exemple sous forme de stick.

Quand la composition utilisée selon l'invention comporte une phase huileuse, celle-ci contient de préférence au moins une huile. Elle peut contenir en outre d'autres corps gras.

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées.

Selon un mode particulier de réalisation de l'invention, la composition contenant les composés anti-pollution est une émulsion eau-dans-huile (E/H) ou huile-dans-eau (H/E). La proportion de la phase huileuse de l'émulsion peut aller de 5 à 80 % en poids, et de préférence de 5 à 50 % en poids par rapport au poids total de la composition. Les huiles, les émulsionnants et les co-émulsionnants utilisés dans la composition sous forme d'émulsion sont choisis parmi ceux classiquement utilisés dans le domaine cosmétique ou dermatologique. L'émulsionnant et le co-émulsionnant sont généralement présents dans la composition, en une proportion allant de 0,3 à 30 % en poids, et de préférence de 0,5 à 20 % en poids par rapport au poids total de la composition. L'émulsion peut, en outre, contenir des vésicules lipidiques.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E).

Pour les émulsions E/H, on peut citer par exemple comme émulsionnants les dimethicone copolyols tels que le mélange de cyclomethicone et de dimethicone copolyol, vendu sous la dénomination « DC 5225 C » par la société Dow Coming, et les alkyl-dimethicone copolyols tels que le Laurylmethicone copolyol vendu sous la dénomination "Dow Corning 5200 Formulation Aid" par la société Dow Corning et le Cetyl dimethicone copolyol vendu sous la dénomination Abil EM 90^{R} par la société Goldschmidt. On peut aussi utiliser comme tensioactif d'émulsions E/H, un organopolysiloxane solide élastomère réticulé comportant au moins un groupement oxyalkyléné, tel que ceux obtenus selon le mode opératoire des exemples 3, 4 et 8 du document US-A-5,412,004 et des exemples du document US-A-5,811,487, notamment le produit de l'exemple 3 (exemple de synthèse) du brevet US-A-5,412,004. et tel que celui commercialisé sous la référence KSG 21 par la société Shin Etsu.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; et leurs mélanges tels que le mélange de stéarate de glycéryle et de stéarate de PEG-40.

De façon connue, la composition cosmétique ou dermatologique de l'invention peut contenir également des adjuvants habituels dans le domaine cosmétique ou dermatologique, tels que les gélifiants hydrophiles ou lipophiles, les actifs hydrophiles ou lipophiles, les conservateurs, les antioxydants, les solvants, les parfums, les charges, les filtres UV, les bactéricides, les absorbeurs d'odeur, les matières colorantes, les extraits végétaux, les sels. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les sphérules lipidiques.

Comme charges qui peuvent être utilisées dans la composition de l'invention, on peut citer par exemple, outre les pigments, la poudre de silice ; le talc ; les particules de polyamide et notamment celles vendues sous la dénomination ORGASOL par la société Atochem ; les poudres de polyéthylène ; les micro-sphères à base de copolymères acryliques, telles que celles en copolymère diméthacrylate d'éthylène glycol/methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; et leurs mélanges. Ces charges peuvent être présentes dans des quantités allant de 0 à 20 % en poids et de préférence de 1 à 10 % en poids par rapport au poids total de la composition.

Selon un mode préféré de réalisation de l'invention, la composition utilisée selon l'invention contient au moins un filtre UV (ou filtre solaire) qui peut être un filtre chimique ou un filtre physique ou un mélange de tels filtres.
A titre d'illustration et de façon non limitative, on peut citer les familles suivantes (les noms correspondent à la nomenclature CTFA des filtres) :
les anthranilates, en particulier l'anthranilate de menthyle ; les benzophénones, en particulier la benzophénone-1, la benzophénone-3, la benzophénone-5, la benzophénone-6, la benzophénone-8, la benzophénone-9, la benzophénone-12, et préférentiellement la Benzophénone-2 (Oxybenzone), ou la Benzophénone-4 (Uvinul MS40 disponible chez B.A.S.F.) ; les benzylidènes-camphres, en particulier le 3-benzylidène-camphre, l'acide benzylidènecampho-sulfonique, le benzalkoniumméthosulfate de Camphre, le polyacrylamidométhylbenzylidène camphre, l'acide téréphthalylidène di-camphre sulfonique , et préférentiellement le 4-méthylbenzylidène camphre (Eusolex 6300 disponible chez Merck) ; les benzimidazoles, en particulier le benzimidazilate (Neo Heliopan AP disponible chez Haarmann et Reimer), ou l'acide phénylbenzimidazole sulfonique (Eusolex 232 disponible chez Merck) ; les benzotriazoles, en particulier le drométrizole trisiloxane, ou le méthylène bis-benzotriazolyltétraméthylbutylphénol (Tinosorb M disponible chez Ciba) ; les cinnamates, en particulier le cinoxate, le DEA méthoxycinnamate, le méthylcinnamate de diisopropyle, le glycéryl éthylhexanoate de diméthoxycinnamate, le méthoxycinnamate d'isopropyle, le cinnamate d'isoamyle, et préférentiellement l'éthocrylène (Uvinul N35 disponible chez B.A.S.F.), l'octylméthoxycinnamate (Parsol MCX disponible chez Hoffmann La Roche), ou l'octocrylène (Uvinul 539 disponible chez B.A.S.F.); les dibenzoylméthanes, en particulier le butyl méthoxydibenzoylméthane (Parsol 1789) ; les imidazolines, en particulier l'éthylhexyl diméthoxybenzylidène dioxoimidazoline ; les PABA, en particulier l'éthyl Dihydroxypropyl PABA, l'éthylhexyldiméthyl PABA, le glycéryl PABA, le PABA, le PEG-25 PABA, et préférentiellement la diéthylhexylbutamido-triazone (Uvasorb HEB disponible chez 3V Sigma), l'éthylhexyltriazone (Uvinul T150 disponible chez B.A.S.F.), ou l'éthyl PABA (benzocaïne) ; les salicylates, en particulier le salicyclate de dipropylèneglycol, le salicylate d'éthylhexyle, l'homosalate, ou le TEA salicylate ; les triazines, en particulier l'anisotriazine (Tinosorb S disponible chez Ciba) ; le drometrizole trisiloxane, l'oxyde de zinc et le dioxyde de titane.

Des exemples de filtres UV convenant particulièrement bien à une utilisation dans la présente invention sont :
- le butyl méthoxydibenzoylméthane vendu notamment par la société HOFFMANN-LAROCHE sous la dénomination Parsol 1789,
- l'octocrylène vendu notamment par la société BASF sous la dénomination Uvinul N539,
- l'octyl salicylate vendu notamment par la société HAARMAN-REIMER sous la dénomination Neo Heliopan OS,
- l'octyl méthoxycinnamate vendu notamment par la société HOFFMANN-LAROCHE sous la dénomination Parsol MCX,
- l'acide phénylbenzimidazole sulfonique vendu notamment par la société MERCK sous la dénomination Eusolex 232,
- les oxybenzones telles que les benzophénones-3, -4 ou -5,
- les silicones benzotriazoles et en particulier le drométrizole trisiloxane,
- l'acide téréphtalylidène di-camphre sulfonique, et
- les oxydes de titane ou de zinc, sous forme de micro- ou nanoparticules éventuellement enrobées.

On utilise de préférence comme filtre dans la composition de l'invention la benzophénone-3, l'acide téréphtalylidène di-camphre sulfonique, l'octyl méthoxycinnamate, l'acide phénylbenzimidazole sulfonique, le drometrizole trisiloxane, le 4-methylbenzylidène camphre, l'anizotriazine, l'octocrylène, le butylmethoxydibenzoyl methane, l'oxyde de zinc et/ou le dioxyde de titane.

La quantité de filtres dépend de l'utilisation finale souhaitée. Elle peut aller par exemple de 1 à 20% en poids et mieux de 2 à 10% en poids par rapport au poids total de la composition.

Selon un autre mode préféré de réalisation selon l'invention, la composition utilisée contient en outre un actif antioxydant (par exemple la vitamine E).

Les compositions utilisées selon l'invention peuvent notamment constituer un produit de soin et/ou de maquillage des matières kératiniques, et notamment de la peau. Elles peuvent être utilisées notamment pour protéger l'organisme, en particulier les matières kératiniques, contre les effets de la pollution, notamment pour améliorer la respiration cellulaire et/ou diminuer la desquamation et/ou pour éviter de ternir ou de salir les matières kératiniques, et notamment la peau.

Ainsi, un autre objet de l'invention consiste en un procédé de traitement cosmétique en vue de protéger les matières kératiniques contre les effets de la pollution, comprenant l'application sur les matières kératiniques d'une composition contenant, dans un milieu physiologiquement acceptable, un complexe de photoprotecteurs cellulaires tel que défini précédemment.

L'invention a aussi pour objet un procédé de traitement cosmétique des matières kératiniques en vue d'améliorer leur respiration cellulaire et/ou de diminuer leur desquamation et/ou d'éviter de les ternir et/ou de les salir, comprenant l'application sur les matières kératiniques d'une composition contenant, dans un milieu physiologiquement acceptable, un complexe de photoprotecteurs celleulaires tel que défini précédemment.

Les exemples qui suivent servent à illustrer l'invention sans toutefois présenter un caractère limitatif. Les noms sont, selon le cas, en noms chimiques ou noms CTFA (International Cosmetic Ingredient Dictionary and Handbook) et les quantités en pourcentage en poids sauf mention contraire.

### Exemple 1 : Mise en évidence de l'effet protecteur du complexe de photoprotecteurs cellulaires naturels sur kératinocytes humains in vitro vis à vis d'un polluant gazeux représentatif: l'ozone.

### • RESUME DU PROTOCOLE EXPERIMENTAL

### Principe :

La méthode utilisée (test au 2,7-DiCHloroFluoresceine diacetate (LeBel C.P. et coll., 1992, Zhu H. et coll., 1994)) permet de réaliser en temps réel une mesure globale de stress oxydatif. Les peroxydes lipidiques ainsi que le peroxyde d'hydrogène générés lors d'un processus d'oxydation des lipides sont capables en présence de peroxydases d'oxyder la 2,7-dichlorofluoresceine (forme réduite non fluorescente). La 2,7-dichlorofluoresceine formée par oxydation est un composé fluorescent dont les longueurs d'onde d'excitation et d'émission sont de 480 nm et 530 nm, respectivement.

### Ensemencement des cellules (kératinocytes humains immortalisés) :

Les cellules DK7-NR (NESTEC) sont ensemencées dans des plaques 48 puits à raison de 5000 cellules / cm² dans 500µl de milieu de culture (milieu défini sans sérum de veau, Biofluids ) jusqu'à confluence.
Elles sont ensuite incubées en étuve 37°C, 5% CO2, atmosphère humide.

### Traitement des kératinocytes :

La substance à tester est préparée dans le milieu de culture à une concentration correspondant à la moitié de la concentration maximale non cytotoxique. Puis elle est mise en contact des cellules pendant 18 heures. Avant l'exposition à l'ozone, les cellules sont lavées.
Les cellules sont alors mises en contact avec le DCHF (320 µM dans du PBS), pendant 30 minutes à 37°C.

### Exposition à l'Ozone:

Après rinçage et élimination du marqueur non incorporé, les cellules sont exposées à l'ozone pendant différents temps (en présence du protecteur) dans un incubateur (37°C, atmosphère humide), concentration en ozone: 10 ppm
L'apparition de DCFH (fluorescence excitation:485, émission: 530) est mesurée après les différents temps d'exposition : 0, 5, 10, 20 minutes.
L'augmentation de la fluorescence de chaque puits (liée au stress oxydant induit) est suivie en cinétique sur un spectrofluorimètre. Les résultats en unités de fluorescence sont exprimés par rapport aux témoins non protégés.

### • RESULTATS

Les cellules ont été mises en contact avec un complexe de photoprotecteurs cellulaires naturels (Photonyl®) à 0,025 % pendant 18 heures, puis à 0,05 % pendant le temps de contact avec l'ozone (1 ppm). La forte diminution du stress oxydant mesurée en présence du produit met en évidence l'effet protecteur significatif du complexe de photoprotecteurs cellulaires naturels. Cet effet est stable jusqu'à 20 minutes d'exposition à l'ozone, la diminution du stress oxydant induit par le gaz étant comprise entre 63.4 % et 62.3% par rapport au témoin. L'effet protecteur diminue ensuite en fonction du temps tout en restant à une valeur non négligeable, comme le montre le tableau ci-dessous :

| **Temps de Contact (mn)** | **% de diminution du stress oxydant (1)** |
|---|---|
| 5 | 36,6 |
| 10 | 34,7 |
| 20 | 37,7 |
| 30 | 42,7 |
| 40 | 51,9 |
| (1) : moyenne obtenue sur quatre expériences | |

### Exemples de composition

### Exemple 2 : Emulsion H/E

| **Phase A (grasse) :** | |
|---|---|
| Monodiglycérylstéarate | 3 g |
| Huile de vaseline | 3 g |
| Alcool cétylique | 5 g |
| | |

| **Phase B (Phase aqueuse) :** | |
|---|---|
| Polyéthylène glycol oxyéthyléné par 50 moles d'oxyde d'éthylène | 3 g |
| Eau qsp | 100 g |

| **Phase C** | |
|---|---|
| Photonyl® | 5 g |
| Eau | 10 g |

### Mode opératoire :

La phase grasse (A) et la phase aqueuse (B) sont préparées séparément et chauffées à 70°C. La phase grasse est versée dans la phase aqueuse sous agitation. L'émulsification est prolongée pendant 10 minutes, puis on refroidit lentement en agitant à une température de 40°. La phase C est ajoutée et on poursuit le refroidissement. On obtient une crème apte à être appliquée sur la peau pour la protéger contre les effets de la pollution.

### Exemple 3 :

De façon classique on formule la composition suivante :

| | |
|---|---|
| Complexe de photoprotecteurs cellulaires (Photonyl®) | 1 g |
| Octylpalmitate | 10 g |
| glycérylisostéarate | 4 g |
| Huile de vaseline | 20 g |
| Sorbitol | 2 g |
| Vitamine E | 1 g |
| Glycérol | 3 g |
| Eau qsp | 100 g |

### Exemple 4 : Gel

| **Phase A :** | |
|---|---|
| Eau | 10 g |
| Photonyl® | 5 g |
| | |

| **Phase B :** | |
|---|---|
| Hydroxypropyle cellulose | 0.10 g |
| Carbopol ULTREZ 10 | 0.25 g |
| Polyéthylène glycol oxyéthyléné par 50 moles d'oxyde d'éthylène | 3 g |
| Eau qsp | 100 g |
| | |

| **Phase C** | |
|---|---|
| Triéthanolamine qsp pH 7 | |
| | |

| **Phase D** | |
|---|---|
| Timiron (Mica recouvert de titane) | 0.5% |

### Mode opératoire :

Les gélifiants de la phase B sont dispersés dans la phase A sous agitation vive. Le mélange obtenu est neutralisé avec la phase C. Enfin la phase D est dispersée sous agitation lente. On obtient un gel apte à être appliqué sur la peau pour la protéger contre les effets de la pollution.

## Revendications

1. Utilisation cosmétique d'un complexe de photoprotecteurs cellulaires, constitué d'un mélange comprenant au moins un acide aminé et au moins un nucléoside et/ou un nucléotide, comme agent cosmétique anti-pollution, dans une composition pour application topique sur les matières kératiniques.

2. Utilisation d'un complexe de photoprotecteurs cellulaires, constitué d'un mélange comprenant au moins un acide aminé et au moins un nucléoside et/ou un nucléotide, pour la préparation d'une composition à application topique destinée à protéger les matières kératiniques contre les effets nocifs de la pollution.

3. Utilisation selon la revendication 1 ou 2 **caractérisée par le fait que** le complexe de photoprotecteurs cellulaires utilisé est d'origine naturelle.

4. Utilisation selon l'une quelconque des revendications précédentes, **caractérisée par le fait que** la quantité de complexe de photoprotecteurs cellulaires va de 0,01 à 10 % en poids par rapport au poids total de la composition.

5. Utilisation selon la revendication 4, **caractérisée par le fait que** la quantité de complexe de photoprotecteurs cellulaires va de 0,05 à 2 % en poids par rapport au poids total de la composition.

6. Utilisation selon l'une quelconque des revendications précédentes, **caractérisée par le fait que** le complexe de photoprotecteurs cellulaires comprend un mélange d'arginine, de mannitol, de chlorhydrate de pyridoxine, de chlorhydrate d'histidine, d'ARN hydrolysé, de triphosphate d'adénosine et de sodium, de phénylalanine, et de tyrosine.

7. Utilisation selon l'une quelconque des revendications précédentes, **caractérisée en ce que** la composition contient en outre au moins un actif antioxydant.

8. Utilisation selon l'une quelconque des revendications précédentes, **caractérisée en ce que** la composition contient en outre au moins un filtre UV.

9. Procédé de traitement cosmétique en vue de protéger les matières kératiniques contre les effets de la pollution, comprenant l'application sur les matières kératiniques d'une composition contenant, dans un milieu physiologiquement acceptable, un complexe de photoprotecteurs cellulaires constitué d'un mélange comprenant au moins un acide aminé et au moins un nucléoside et/ou un nucléotide.

10. Procédé de traitement cosmétique des matières kératiniques en vue d'améliorer leur respiration cellulaire et/ou de diminuer leur desquamation et/ou d'éviter de les ternir et/ou de les salir, comprenant l'application sur les matières kératiniques d'une composition contenant, dans un milieu physiologiquement acceptable, un complexe de photoprotecteurs cellulaires constitué d'un mélange comprenant au moins un acide aminé et au moins un nucléoside et/ou un nucléotide.

11. Procédé selon la revendication 9 ou 10, **caractérisé par le fait que** le complexe de photoprotecteurs cellulaires utilisé est d'origine naturelle.

12. Procédé selon la revendication 9 à 11, **caractérisé par le fait que** la composition contient en outre au moins un actif antioxydant.

13. Procédé selon la revendication 9 à 11, **caractérisé par le fait que** la composition contient en outre au moins filtre UV.

14. Procédé selon l'une quelconque des revendications 9 à 13, **caractérisé par le fait que** la matière kératinique est la peau.
